# EUROPEAN PATENT SPECIFICATION

(11) **EP 0 478 184 B1**
(45) Date of publication and mention of the grant of the patent: **19.11.1997**
(21) Application number: 91308371.3
(22) Date of filing: 13.09.1991
(51) Int. Cl.: C08G 65/00, C08G 65/32, C07C 59/125

(54) **Oxidation of polyethers**
Oxydation von Polyethern
Oxydation de polyéthers

(30) Priority: 27.09.1990 GB 9021091
(43) Date of publication of application: 01.04.1992
(73) Proprietor: BP Chemicals Limited, London EC2M 7BA (GB)
(72) Inventor: Attfield, Michael James Adibis, Redhill, Surrey RH1 1RW (GB); Pearce, Andrew, Hedon, Hull Hu12 8DS (GB); Slater, Barry, Hedon, Hull Hu12 8DS (GB)
(74) Representative: Denbigh, Keith Warwick

(56) References cited:
- WO-A-84/04754
- GB-A- 971 173
- US-A- 3 330 795
- US-A- 3 560 574
- US-A- 3 931 024
- DATABASE WPI Week 9027, Derwent Publications Ltd., London, GB; AN 90-204892
- TETRAHEDRON LETTERS vol. 31, no. 38, 1990, pages 5479-5482 T.FUJISAWA ET AL.

## Description

The present invention relates to a process for the production of an additive suitable for incorporation into a lubricating oil or a fuel by the reaction of an aliphatic polyamine with a polymer having keto-carbonyl functional groups.

Oxidised polymers, that is polymers having oxygen-containing functional groups, and in particular keto-carbonyl functional groups, are potentially valuable industrial products because they are readily convertible by reaction of the oxidised moieties associated therewith to products useful, amongst other applications, as fuel and lubricating oil additives, for example as dispersants, and detergents. Perhaps the most reported polymer oxidation is that of the polyolefins, for example polyisobutene and polyethylene.

The oxidation and subsequent amination of polyisobutenes has been described in the patent literature in, for example, US Patent No. 3,931,024, which discloses that a highly effective dispersant for a lubricating oil or a fuel can be prepared by the noncatalysed air-oxidation of a polyolefin or of a halogenated polyolefin to form an oxygen-containing material. This material is then reacted with an aliphatic polyamine under conditions that cause the formation of an addition product which differs chemically from the Schiff bases of the prior art.

In GB-A-959362 there is described the oxidation of polyethylene waxes.

Our copending EP-A-0271261 discloses a process for the production of an oxidised polyisobutene which process comprises passing through a column of the polyisobutene maintained at a temperature in the range from 140 to 200°C and at atmospheric or elevated total pressure a molecular oxygen-containing gaseous oxidant at a gas flow rate greater than 10 litres cm⁻²h⁻¹ measured at the operating pressure.

The oxidation of polymers containing -0- bonds in the backbone chain is less common. JP-A-2198 641 describes the preparation of a carboxylic acid salt by oxidising a compound of the formula RO(ethylene oxide)ₙH with oxygen in an aqueous solvent at a pH above 7, a temperature of 30 to 100°C and a pressure of below 10 atmospheres in the presence of a catalyst consisting of (i) Pt and/or Pd, with (ii) Se, Te, Sb, Sn, Bi and/or Pb, the atomic ratio of (ii) to (i) being 0.01-2.0.

GB-A-971173 discloses ketonic-ended polyethers of the general formula:- 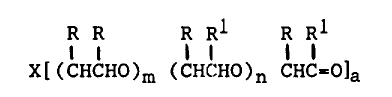 wherein X stands for a monovalent or polyvalent radical derived from a compound X(H)ₐ in which a is the number of hydrogen atoms reactive with a cyclic oxide, R stands for hydrogen or a hydrocarbon radical, R¹ stands for a hydrocarbon radical, n stands for an integer of at least one and m stands for zero or an integer. The polyethers may be prepared by the oxidation of suitable polyethers having secondary hydroxyl end groups.

JP-A-2135224 discloses that polyketones (PAPK) are produced by the reaction of polyoxyalkylenepolyols containing secondary OH groups in the molecule with dehydrogenation catalysts.

US-A-3,560,574 discloses compounds of the formula:- 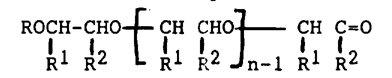 wherein R¹ and R² are hydrogen or an organic radical, which can include inert substituents, and their preparation by the trihydrocarbyl phosphine-catalysed reaction of epoxides and monohydric alcohols.

It has now been found that a polyether having keto-carbonyl functional groups is convertible by reaction of the keto-carbonyl groups with an aliphatic polyamine to an additive suitable for lubricating oil and fuel compositions.

According to the present invention there is provided a process for the production of an additive suitable for incorporation into a lubricating oil or a fuel which process comprises reacting an aliphatic polyamine with a compound of the formula (I):- 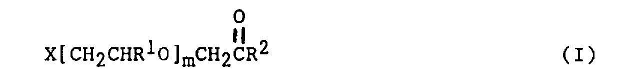 wherein
X is a group derived from a nucleophile;
R¹ is hydrogen, alkyl or aryl;
R² is alkyl or aryl;
m is a number from 1 to 40; and
when m is greater than one each [CH₂CHR¹O] unit is the same or different provided that when R¹ is hydrogen the [CH₂CH₂O] units comprise no more than 50% by weight of the total number of [CH₂CHR¹O] units.

The group X in the compounds of formula (I) is a group derived from a nucleophile. X can suitably be -OH, -SH, -OR³, -SR³ (where R³ = hydrocarbyl, for example alkyl, alkenyl, aryl, alkaryl or aralkyl, preferably alkaryl; where R³ is an alkyl group it is suitably a C₁-C₅₀ alkyl, preferably C₁-C₃₀, more preferably C₁-C₂₀), -NR⁴R⁵ (where R⁴, R⁵ are independently H, or a hydrocarbyl group, preferably a C₁-C₂₀, more preferably C₁-C₁₀ alkyl group, for example methyl or ethyl) or alkanoate (for example acetate). The group X is preferably an alkyl substituted phenate group for example dodecylphenate -OC₆H₄(C₁₂H₂₅).

In the formula (I), R¹ and R² are independently alkyl or aryl. Alternatively R¹ can be H. Where R¹ or R² is an alkyl group it is suitably a C₁-C₃₀ alkyl group, preferably C₁-C₂₀, more preferably C₁-C₁₀, suitably C₁-C₃, for example methyl or ethyl; where R¹ or R² is an aryl group it is preferably phenyl. R¹ and R² can be the same or different, preferably the same. m in the formula I is a number from 1 to 40, preferably 10 to 30, more preferably 15 to 25. Although each molecule will have an integral value of m, any compound of the present invention will comprise molecules of varying sizes and values of m, therefore m may have a value that is not an integer. Compounds of the formula I comprise a backbone of units of the formula [CH₂CHR¹O] where each of the units are the same or different. Where the units are different, the backbone may contain two or more different types of [CH₂CHR¹O] units. Where R¹ is hydrogen, the [CH₂CH₂O] units comprise no more than 50% by weight of the compound, preferably no more than 30%, more preferably no more than 15%.

The compound of formula (I) is preferably obtained by oxidising a compound of the formula (II):-

X[CH₂CHR¹O]ₘCH₂CHR²(OH) (II)

wherein X, R¹ R² and m are all as defined hereinbefore in relation to the compound of formula (I), provided that when R¹ is H, the [CH₂CH₂O] units comprise no more than 50% by weight of the total number of [CH₂CHR¹O] units.

The starting material for preparing the compound of formula (I) is preferably a compound of formula (II) and is preferably a polyether obtained by the anionic polymerisation of alkylene or aryl-alkylene epoxide initiated by a suitable nucleophile. Polyethers which may be used in the process of the invention are the polyalkylene polyethers, and polyaryl-substituted alkylene polyethers, for example polypropylene oxide, polybutylene oxide, and polystyrene oxide. Alternatively, the polyethers may comprise two or more different oxyalkylene units for example a mixed polyethylene oxide/polypropylene oxide based polyether. Types of polyether which may be used in the process of the invention are, for example polyether amines (X = NR⁴R⁵) or polyether polyols (X = OH). Examples of suitable polyethers useful in the process of the invention are those having the formula:- 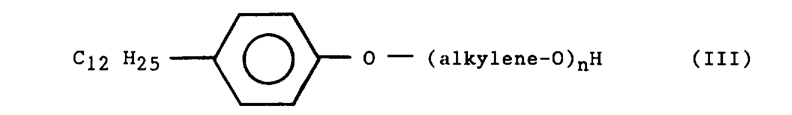 wherein the alkylene group is propylene or butylene and n is an integer having a value of about 25. Polyethers of the formula (III) are commercially available.

The oxidation of the polyether may be accomplished in a variety of ways, for example by treatment with dichromate, potassium permanganate, silver (I) complexes (e.g. Tollens reagent), copper (I) or (II) complexes (e.g. Fehlings reagent) or a peroxide or hydroperoxide (e.g. H₂O₂), preferably by treatment with dichromate.

Where dichromate is used it is preferably used as chromic acid. The dichromate to polyether molar ratio is preferably in the range 1:1 to 4:1, more preferably 1.5:1 to 2.5:1 for example 2:1. Where chromate is used as an oxidant, the reaction temperature is preferably in the range 25 - 100°C, more preferably in the range 35 - 65°C.

The oxidation can also be carried out by catalytic dehydrogenation using supported metal oxide e.g. copper-chromium oxides on celite. Alternatively, the oxidation can be carried out using a molecular oxygen-containing gas.

Where a molecular oxygen-containing gas is used it should be of a composition such as to remain outside the explosive limits of any light hydrocarbons formed during the oxidation. A suitable molecular oxygen-containing gas for this purpose is oxygen diluted with inert gas, for example nitrogen. Suitably in such a mixture the oxygen concentration is in the range from 2 to 20% by volume. Air or other molecular oxygen-containing gases may also be used, subject to the aforementioned proviso. It is preferred to recycle the gaseous oxidant after passage through the polymer since only a small proportion of the available oxygen may be absorbed per pass through the column of reactant.

The oxidation using a molecular oxygen-containing gas may suitably be accomplished by passing the gas through a liquid column of the polyether maintained at a temperature in the range from 50 to 150°C. The liquid column of polymer may suitably be contained within a tubular reactor. A preferred form of tubular reactor is a draft tube reactor, i.e. a reactor which comprises a closed vessel having at least one inlet and at least one outlet, a partition dividing the vessel into two zones which intercommunicate above and below the partition and means for admitting gas in a finely divided state into the lower part of one of the zones.

The foregoing is merely illustrative of one suitable mode of operation. There are many other ways of practising the process of the invention.

It is preferred to add to the polyether before oxidation a portion of a polyether oxidate. This can lead to the advantage that the initial oxidation rate may be increased.

The product resulting from the process comprises a ketone derivative of formula (I) but may also comprise ester derivatives of the polyether. Typically, oxidation using a molecular oxygen-containing gas of a polybutylene oxide derived from the base catalysed polymerisation of butylene oxide with dodecylphenol produces an ethyl ketone derivative, a formate ester derivative and a propionate ester derivative of the polyether. Oxidation of a similar polymer in which butylene oxide is replaced by propylene oxide produces a formate ester derivative, an acetate ester derivative and a methyl ketone derivative of the polyether. In addition to the chemical functionalisation of the polyether physical degradation occurs.

Where a molecular oxygen-containing gas is used it is preferred that there is added to the polyether a soluble transition metal compound.

It has been observed that there is a minimum temperature at which molecular oxygen uptake occurs, which temperature depends amongst other factors on the nature of the polyether. It has been found that for any particular polyether the minimum temperature required for oxygen uptake to occur can be reduced by the addition of a soluble transition metal compound. Moreover, addition of the transition metal compound can also increase the overall conversion of the oxidation reaction under identical conditions.

Suitably the transition metal of the transition metal compound may be for example copper, chromium, cobalt or iron. Suitably the compound may be a salt or a complex of the transition metal. It is believed that it is principally the metallic moiety of the compounds which influences its catalytic effect, rather than the non-metallic moiety. A suitable metal compound is the acetylacetonate complex of the metal.

Oxidation conditions comprise an elevated temperature, suitably in the range from 50 to 150°C and either atmospheric, subatmospheric or superatmospheric pressure. Within the aforesaid temperature range, for each polyether there is a critical temperature below which oxidation does not appear to occur. It is well within the ordinary skill in the art to determine this temperature.

Preferably the polyether is in the liquid phase. Using polyethers of a molecular weight such that they are liquid under the oxidation conditions the liquid phase may be provided by the liquid polyether. Polyethers which are solid under the oxidation conditions may suitably be used in the form of either a dispersion or a solution in a suitable high-boiling liquid which is inert under the oxidation conditions. Liquid polyethers too, if desired, may be used as either a dispersion or a solution.

The production of an additive suitable for incorporation into a lubricating oil or fuel preferably comprises reacting a keto-functionalised polyether obtained by a process as hereinbefore described with an aliphatic polyamine employing a mole ratio of polyamine to polyether of from about 0.2 to about 2.5 moles of polyamine per mole of the polyether to form an aminated polyether product.

The oxidation product may be used without any intervening purification as the keto-functionalised polyether starting material for the amination process. The aliphatic polyamine may suitably be an alkylene polyamine having the general formula (IV):

NH₂(CH₂)_{q}-[NH(CH₂)_{q}]ᵣNH₂ (IV)

wherein q is an integer from 2 tc 4 and r is zero or an integer from 1 to 10. Specific compounds falling within the aforesaid formula (IV) include ethylene diamine, diethylene triamine, triethylene tetramine, tetraethylene pentamine, propylene diamine, dipropylenetriamine, and the like. A preferred alkylene polyamine is diethylene triamine.

Alternatively, the polyamine may be of the formula (V): 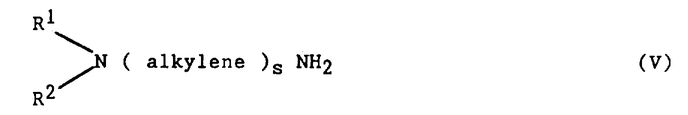 wherein R¹ and R² are independently hydrogen or alkyl groups, for example a C₁-C₁₀ alkyl group preferably methyl, ethyl, or propyl and s is an integer from 1 to 6, preferably 2 to 4.

A suitable polyamine of the aforesaid formula (V) is Me₂N(CH₂)₃NH₂.

Further details of the amination process may be found in US Patent No. 3,931,024. Typically, the temperature of the amination reaction may be in the range from 60 to 250°C.

It is preferred to hydrogenate the amination product before use as an additive. Hydrogenation is desirable primarily to convert imines in the amination product to amines. Hydrogenation may suitably be accomplished by, for example, treatment with an alkali metal borohydride, eg sodium borohydride. Alternatively, the amination product may be reacted with formic acid. In a further alternative the amination product may be catalytically hydrogenated, suitably using as catalyst a supported transition metal, preferably a supported platinum group metal, for example palladium supported on carbon or alumina. Other hydrogenation techniques known in the art may also be used. The present invention also provides a finished lubricating oil composition comprising a major proportion of a lubricating oil and a minor proportion of an additive as hereinbefore described. Such a lubricating oil composition will typically comprise 0.1 to 10%w/w of the additive, preferably 0.5 to 7, more preferably 1 to 6%w/w.

The lubricating oil may suitably be any oil of lubricating viscosity and may be either natural or synthetic.

In a further embodiment the invention also provides a fuel composition comprising a major proportion of a fuel and a minor proportion of the additive prepared by the process as hereinbefore described. Such a fuel composition will typically comprise 5-5,000ppm, preferably 50-1,000 ppm (by weight) of the additive.

The fuel may suitably be an internal combustion engine fuel. A preferred fuel is one suitable for use in spark ignition engines, for example a motor gasoline. Alternatively, the fuel may be a fuel suitable for use in compression ignition engines, for example a diesel fuel.

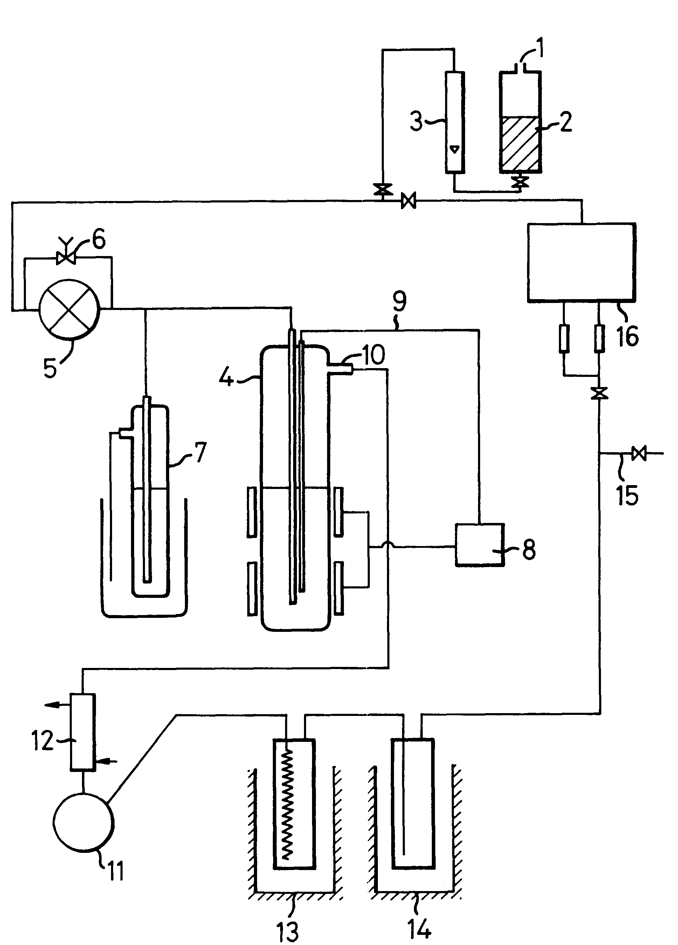

The invention will now be further illustrated by reference to the following Examples and the accompanying Figure which takes the form of a flowsheet.

The plant used in examples 1-14 comprises an inlet (1) fitted with a silica gel drying column (2) and a rotameter (3) which is connected to the reactor (4) via a pump (5). The pump (5) can be by-passed by means of a flow control valve (6). A mercury blow off (7) is fitted in between the pump (5) and reactor (4). The reactor (4) is fitted with a eurotherm heatcontroller (8) attached to a thermocouple (9). Gaseous components can be vented from an outlet (10) through a catch-pot (11) fitted with a condenser (12) and two cold traps (13, 14) to a large-bore vent (15) or alternatively can be recycled through the oxygen meter (16). All lines are made of PTFE except for the dry air line from the rotameter.

The plant shown in the Figure may be operated in either of two ways as follows:-
(i) once-through operation:- Air is taken in through the silica gel drying column (2) and thereafter through the rotameter (3) before being pumped through the reactor (4) and traps (13, 14) and exiting out of the vent (15) before the oxygen meter (16).
(ii) recycle operation:- The system is filled up with all valves open. The vent (15) and inlet (1) are then closed off and air is circulated through the oxygen meter (16). It is therefore possible to monitor oxygen depletion versus time for a specific flow rate.

### Preparation of polyether having keto-carbonyl functional groups Preparation 1

246.7g of PC1362 (a polybutylene oxide derived from the base catalysed polymerisation of an average of 25 moles of butylene oxide with dodecylphenol) was weighed into the oxidation reactor. The air pump was switched on and nitrogen was pumped through the system in the once-through operation mode. The reactor tube was heated up to 125°C and then air was sparged through the system at a rate of 372 litres/hour for 3 hours. (This flow rate was used for all 35 subsequent oxidation reactions.)

Less than 0.5g of low molecular weight condensate was collected in the cold traps.

The product was transferred to a 500ml 3 neck round bottomed flask in which it was vacuum stripped at 150°C for 30 minutes to remove any low boiling fractions.

### Preparation 2

233.7g of PC1362 was oxidised under identical conditions to those employed in Preparation 1 except that air was sparged through the heated polyether for 6 hours.

11.9g of low molecular weight condensate was collected in the cold traps.

The product was vacuum stripped as per Preparation 1.

### Analytical Data for Preparations 1 and 2

The infra red spectra of both Preparations showed a carbonyl absorbance centred at 1728 cm⁻¹ not present in PC1362. the intensity of the absorbance was stronger for Preparation 2, indicating a greater degree of oxidation.

### Gel Permeation Chromatography Data

| SAMPLE | Mn | Mw | Mw/Mn |
|---|---|---|---|
| PC1362 | 1950 | 2210 | 1.1 |
| Preparation 1 | 1620 | 1810 | 1.1 |
| Preparation 2 | 960 | 1430 | 1.5 |

### ¹H Nuclear Magnetic Resonance Data

| SAMPLE | % SELECTIVITIES | | | % CONVERSION |
|---|---|---|---|---|
| | Ethyl Ketone | Formate Ester | Propionate Ester | |
| Preparation 1 | 35 | 45 | 20 | 15 |
| Preparation 2 | 37 | 39 | 25 | 104 |

The conversion figure for Preparation 2 implies that more than one oxidative attack per polymer molecule has occurred.

### Preparation 3

The reactor tube was filled with 237.0g of PC1572 (which is the polypropylene oxide analogue of PC1362 i.e. dodecylphenol and 25 moles of propylene oxide). The reactor tube was heated to 100°C whilst sparging with nitrogen in the once-through mode. The system was then filled with air and switched to recycle operation. The oxygen content of the system was observed to fall from 20% to 2% whereupon the system was refilled with air via the silica gel tube inlet. Another three oxygen depletions of the system were carried out. The total time for all four depletions was 7 hours 33 minutes. 2.14g of low molecular weight condensate was collected.

The product was vacuum stripped at 150°C for 30 minutes using a pressure of 5.08cm (2") Hg.

### Preparation 4

The reactor tube was filled with 239.1g of PC1572 and the procedure used in Preparation 3 was repeated except that 8 depletions of the oxygen content of the system from 20% to 2% were carried out. The time taken for these depletions was 12 hours 34 minutes. 5.46g of low molecular weight condensate was collected.

The product was vacuum stripped under the same conditions as Preparation 3.

### Preparation 5

The reactor tube was filled with 237.2g of PC1572 and 12 depletions of the oxygen content of the system were carried out in the same way as Preparation 3. The time taken for these depletions was 29 hours 26 minutes. 11.29g of low molecular weight condensate was collected.
The product was vacuum stripped under the same conditions as Preparation 3.

### Preparation 6

The reactor tube was filled with 237.7g of PC1572 and 16 depletions of the oxygen content of the system were carried out in the same way as Preparation 3. The time taken for these depletions was 30 hours 57 minutes. 14.63g of low molecular weight condensate was collected.

The product was vacuum stripped under the same conditions as Preparation 3.

### Analytical Data for Preparations 3 to 6

The infra red spectra of all Preparations showed a carbonyl absorbance centred at 1728 cm⁻¹, not present in PC1572. The intensity of the absorbance was sequentially greater for Preparations 3 to 6 indicating an increasing extent of oxidation.

### Gel Permeation Chromatography Data

| SAMPLE | Mn | Mw | Mw/Mn |
|---|---|---|---|
| PC1572 | 1750 | 1840 | 1.0 |
| Preparation 3 | 1300 | 1630 | 1.2 |
| Preparation 4 | 1150 | 1530 | 1.3 |
| Preparation 5 | 890 | 1360 | 1.5 |
| Preparation 6 | 690 | 1240 | 1.8 |

### ¹H Nuclear Magnetic Resonance Data

| SAMPLE | % SELECTIVITIES | | | % CONVERSION |
|---|---|---|---|---|
| | Formate Ester | Acetate Ester | Methyl Ketone | |
| PC1572 | 100 | 0 | 0 | 3 |
| Preparation 3 | 57 | 16 | 27 | 24 |
| Preparation 4 | 50 | 19 | 31 | 38 |
| Preparation 5 | 50 | 23 | 27 | 59 |
| Preparation 6 | 51 | 22 | 27 | 68 |

### Preparation 7

The reactor tube was filled with 257.6g of polyether CP-1 (available from BP Chemicals) which is a glycerol started polyethylene oxide/polypropylene oxide. The same procedure of Preparation 3 was used to oxidise the sample for 6 oxygen depletions of the system. This took 11 hours 10 minutes and 9.04g of low molecular weight condensate was collected.

The infra red spectra of the product showed a carbonyl absorbance at 1728 cm⁻¹ not present in CP-1. This absorbance was of similar shape to those observed for Preparations 3 - 6.

Gel permeation chromatography showed that Mn had dropped from 4110 to 2060, Mw had dropped from 4240 to 3100 and the dispersity Mw/Mn had increased from 1.0 to 1.5.

### Preparations 8 - 12

Approximately 230g of PC1362 was weighed into the oxidation reactor tube along with 1% w/w of a metal acetylacetonate salt. It was then heated to 80°C with nitrogen circulating through the system before air was admitted. It was then returned to recycle mode and the temperature was gradually raised until a significant rate of oxygen uptake was observed on the oxygen meter. Once this temperature had been recorded the reactor was heated to 125°C whereupon air was sparged through the reactor in the 'once through' operation mode for 3 hours giving a total reaction time of 3 hours 20 minutes.

The metal salts used for Preparations 8-11 were Fe(acac)₃, Co(acac)₃, Cu(acac)₂ and Cr(acac)₃ respectively. A comparative uncatalysed experiment (Preparation 12) was also performed.

### Analytical Data for Preparations 8 - 12

The infra red spectrum of the product from Preparations 8-11 showed a carbonyl absorbance centred at 1728 cm⁻¹ which was similar in profile to those observed in Preparations 1-2. The intensity of this peak decreased in the order Preparation 8,9,10 and 11. Preparation 12 gave no substantial carbonyl peaks.

| PREP. | METAL SALT | MINIMUM TEMPERATURE FOR OXYGEN UPTAKE (°C) | CARBONYL CONTENT (% wt/wt acetone equivalents) |
|---|---|---|---|
| 8 | Fe(acac)₃ | 80-90 | 2.15 |
| 9 | Co(acac)₃ | 80-85 | 1.30 |
| 10 | Cu(acac)₂ | 80 | 0.82 |
| 11 | Cr(acac)₃ | 85-90 | 0.88 |
| 12 | None | 120 | 0.10 |

It is evident that Fe(acac)₃, Co(acac)₃, Cu(acac)₂ and Cr(acac)₃ all facilitate the oxidation reaction by reducing the minimum process temperature and increasing the overall ketone yield under identical reaction conditions.

### Preparation 13

236.3g of PC1362 and 2.38g of Fe(acac)₃ were weighed into the reactor tube and the tube was heated to 60°C under a sparge of nitrogen. The system was then filled with air which was recycled. A slow uptake of oxygen was observed which became more rapid as the temperature was raised. Once the temperature had reached 90°C the polyether was oxidised with air in the once-through mode for 3 hours giving a total reaction time of 3 hours 20 minutes.

The product showed a similar infra red carbonyl absorption as Preparation 8 but of less intensity as would be expected. The carbonyl content was 1.80% w/w acetone equivalents. This experiment demonstrates that the presence of Fe(acac)₃ enables the oxidation to be carried out at temperatures as much as 30°C lower than in its absence.

### Preparation 14

200g of the polyether PC1362 (dodecylphenol started polybutylene oxide -25 moles butylene oxide) was dissolved in 200ml 40-60 petroleum ether in a 1 litre 3 necked round bottomed flask fitted with overhead mechanical stirrer, water cooled condenser and pressure equalising dropping funnel. 22.84g (2 fold excess relative to PC1362) of solid sodium dichromate dihydrate was dissolved in 306ml of 2N sulphuric acid and this solution was charged to the dropping funnel. With vigorous stirring and under a nitrogen blanket the dichromate solution was added dropwise to the polyether solution over an interval of 30 minutes. The flask was then heated to 45°C with vigorous stirring maintained whereupon the pet ether refluxed into the condenser. This temperature was maintained for 6 hours before the heat was removed and the flask stirred for a further 16 hours at ambient temperature. The contents of the flask were poured into a separating funnel and allowed to form two layers. The lower aqueous layer was run off and the organic layer was sequentially washed with three equal volume aliquots of distilled water. The organic layer was shaken with anhydrous magnesium sulphate to remove residual water, filtered and the pet ether removed on a rotary evaporator. 170.4g of a clear pale green liquid was recovered.

The ¹H nmr spectrum of this product revealed the presence of a CH₂ adjacent to ketone signal at 2.5 ppm. Integration of this signal and the aromatic proton signal between 6.5-7.5 pm implied that virtually quantitative conversion to the polyether ketone had been achieved.

A previous attempt where an equivalence of sodium dichromate was used and no heat was applied only achieved 47% conversion. Preparations 1 to 14 are not examples according to the present invention because the keto-carbonyl functionalised material is not further reacted with an aliphatic polyamine.

### Amination of the Polyether Ketones

### Example 1

150g of the product from Preparation 1 was stirred with 13.6g (1.5 molar equivalents) of dimethylaminopropylamine in a 500 ml round bottomed flask fitted with thermowell, overhead stirrer and Dean-Stark trap with condenser. The mixture was stirred at ambient temperature for one hour. 120 ml of toluene was added to the flask and the mixture was refluxed for a further 4 hours, with any water produced by the reaction being collected in the Dean-Stark trap.

The product was vacuum stripped at 130°C for one hour to remove the toluene and any unreacted amine.

After cooling a solution of 7.00g sodium borohydride in ethanol was added to the flask. Some mild effervescence due to the liberation of hydrogen was observed. The mixture was stirred for twelve hours at ambient temperature before the ethanol was removed on a rotary evaporator.

This reduced aminated product was dissolved in 150 ml of 40-60 pet ether and extracted with three 150 ml aliquots of distilled water to remove unreacted sodium borohydride and residual unreacted dimethylaminopropylamine. The addition of 10 ml of n-butanol was necessary at each stage of this extraction to break down emulsions. The recovered organic layer was dried with anhydrous magnesium sulphate, filtered and then the pet ether and n-butanol was removed on a rotary evaporator.

The basic nitrogen content was 1.01% and the total nitrogen content was 1.07%.

The product from Example 1 was tested in the Opel Kadett Test. 500ppm of an additive comprising 50% w/w of product from Example 1 and 50% w/w of A260 solvent was added to a base fuel. The base fuel and base fuel containing the additive were tested and the results are given in the Table.

**TABLE**

| Sample | Average Intake Valve Deposit | Average Valve Merit Rating |
|---|---|---|
| Base Fuel Containing 500ppm of additive* | 110 mg/valve | 8.9 |
| Base Fuel (no additive) | 487 mg/valve | 7.4 |

| | | |
|---|---|---|
| * Additive Composition : 50% w/w Product of Example 1, 50% w/w A260 Solvent | | |

### Example 2

220g of the product from Preparation 2 was stirred with 33.7g of dimethylaminopropylamine in a 500 ml round bottomed flask fitted with thermowell, overhead stirrer and Dean-Stark trap with condenser. The mixture was stirred at ambient temperature for one hour during which the colour changed from pale green to amber. This colour change is attributable to the formation of amine oxides from hydroperoxides present within the oxidate. 120 ml of toluene was added to the flask and the mixture was refluxed for a further two hours, with any water produced in the reaction being collected in the Dean-Stark trap.

The product was vacuum stripped at 130°C for one hour to remove the toluene and any unreacted amine.

The infra red spectrum of this material showed the disappearance of the oxidate carbonyl peak at 1728 cm⁻¹ and the appearance of a broad imine/amide peak centred at 1679 cm⁻¹.

The basic nitrogen content of this material was 1.18% and the total nitrogen content was 1.78%.

110.0g of the aminated material was dissolved in 100 ml of ethanol and 5.52g of sodium borohydride was stirred in. Some mild effervescence due to the liberation of hydrogen was observed. The mixture was stirred for twelve hours at ambient temperature.

This reduced aminate product was dissolved in 100 ml of toluene and extracted with three 100 ml aliquots of distilled water to remove unreacted sodium borohydride and any water soluble amides. The addition of 10 ml of n-butanol was necessary at each stage of this extraction to break down emulsions. The toluene was removed on a rotary evaporator.

The infra red spectrum of this product showed in the disappearance of all imine and carbonyl absorbances in the 1800-1620 cm⁻¹ region.

The basic nitrogen content was 0.53% and the total nitrogen content was 0.61%.

The product was gasoline soluble at the 10% level.

## Claims

1. A process for the production of an additive suitable for incorporation into a lubricating oil or a fuel which process comprises reacting an aliphatic polyamine with a compound of the formula (I):- 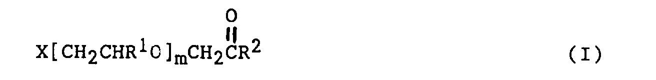 wherein
X is a group derived from a nucleophile;
R¹ is hydrogen, alkyl or aryl;
R² is alkyl or aryl;
m is a number from 1 to 40; and
when m is greater than one each [CH₂CHR¹O] unit is the same or different provided that when R¹ is hydrogen the [CH₂CH₂O] units comprise no more than 50% by weight of the total number of [CH₂CHR¹O] units.

2. A process according to claim 1 wherein in the formula (I) X is OH, SH, OR³, SR³, - NR⁴R⁵ or an alkanoate, wherein R³ is a hydrocarbyl group and R⁴ and R⁵ are independently either hydrogen or a hydrocarbyl group.

3. A process according to either claim 1 or claim 2 wherein R¹ in the formula (I) is either methyl or ethyl.

4. A process according to any one of the preceding claims wherein the compoundd of formula (I) is obtained by oxidising a compound of the formula (II):-
X[CH₂CHR¹O]ₘCH₂CHR²(OH) (II)
wherein X, R¹, R² and m are all as defined in claim 1 provided that when R¹ is H, the [CH₂CH₂O] units comprise no more than 50% by weight of the total number of [CH₂CHR¹O] units.

5. A process according to claim 4 wherein the compound of formula (II) is oxidised by treatment with dichromate.

6. A process according to claim 5 wherein the treatment with dichromate is carried out at a temperature in the range 25 to 100°C.

7. A process according to claim 4 wherein the compound of formula (II) is oxidised by catalytic dehydrogenation.

8. A process according to claim 4 wherein the compound of formula (II) is oxidised by treatment with a molecular oxygen-containing gas.

9. A process according to any one of the preceding claims wherein the ratio of the polyamine to the compound of formula (II) is in the range 0.2 to 2.5:1.

10. A process according to claim 1 wherein a compound of the formula (I) obtained by oxidising a compound of the formula (II) is reacted with an aliphatic polyamine employing a mole ratio of polyamine to polyether of from 0.2 to 2.5 moles of polyamine per mole of polyether.

11. A process according to any one of the preceding claims wherein the polyamine is of the general formula (IV):-
NH₂(CH₂)_{q}[NH(CH₂)_{q}]ᵣNH₂ (IV)
wherein q is an integer from 2 to 4 and r is zero or an integer from 1 to 10.

12. A process as claimed in claim 11 wherein the polyamine is diethylene triamine.

13. A process as claimed in any one of the preceding claims wherein the additive is hydrogenated in a further step.

14. A fuel composition comprising a major proportion of a fuel and a minor amount of an additive prepared by a process as claimed in any one of the preceding claims.

## Patentansprüche

1. Ein Verfahren für die Herstellung eines Additivs, geeignet für die Inkorporierung in ein Schmieröl oder einen Treibstoff, welches Verfahren das Umsetzen eines aliphatischen Polyamins mit einer Verbindung der nachstehenden Formel (I): 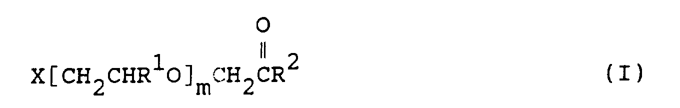 umfaßt, worin X eine von der nukleophilen Verbindung abgeleitete Gruppe bedeutet,
R¹ Wasserstoff, Alkyl oder Aryl ist,
R² Alkyl oder Aryl ist,
m eine ganze Zahl mit einem Wert von 1 bis 40 bedeutet, und
jede [CH₂CHR¹O]-Einheit gleich oder verschieden ist, wenn m größer als eins ist, vorausgesetzt daß, falls R¹ Wasserstoff bedeutet, die [CH₂CH₂O]-Einheiten nicht mehr als 50 Gewichtsprozent der Gesamtzahl der [CH₂CHR¹O]-Einheiten enthalten.

2. Ein Verfahren nach Anspruch 1, worin in der Formel (I) X OH, SH, OR³, SR³, -NR⁴R⁵ oder ein Alkanoat bedeutet, worin R³ eine Hydrocarbylgruppe ist und R⁴ und R⁵, unabhängig, entweder Wasserstoff oder eine Hydrocarbylgruppe sind.

3. Ein Verfahren nach einem der Ansprüche 1 oder 2, worin R¹ in der Formel (I) entweder Methyl oder Ethyl ist.

4. Ein Verfahren nach einem der vorstehenden Ansprüche, worin die Verbindung der Formel (I) durch Oxidieren einer Verbindung der Formel (II):
X[CH₂CHR¹O]ₘCH₂CHR² (OH) (II)
erhalten wird, worin X, R¹, R² und m alle wie in Anspruch 1 definiert sind, vorausgesetzt, daß, falls R¹ H ist, die [CH₂CH₂O]-Einheiten nicht mehr als 50 Gewichtsprozent der Gesamtanzahl der [CH₂CHR¹O]-Einheiten enthalten.

5. Ein Verfahren nach Anspruch 4, worin die Verbindung der Formel (II) durch Behandeln mit Dichromat oxidiert ist.

6. Ein Verfahren nach Anspruch 5, worin die Behandlung mit Dichromat bei einer Temperatur im Bereich von 25° bis 100°C durchgeführt wird.

7. Ein Verfahren nach Anspruch 4, worin die Verbindung der Formel (II) durch katalytische Dehydrierung oxidiert ist.

8. Ein Verfahren nach Anspruch 4, worin die Verbindung der Formel (II) durch Behandeln mit einem molekularen Sauerstoffenthaltenden Gas oxidiert ist.

9. Ein Verfahren nach einem der vorstehenden Ansprüche, worin das Verhältnis des Polyamins zu der Verbindung der Formel (II) im Bereich von 0,2 bis 2,5 : 1 liegt.

10. Ein Verfahren nach Anspruch 1, worin eine Verbindung der Formel (I), erhalten durch Oxidieren einer Verbindung der Formel (II), mit einem aliphatischen Polyamin unter Verwendung eines Molverhältnisses von Polyamin zu Polyether im Bereich von 0,2 bis 2,5 Mol Polyamin pro Mol Polyether, umgesetzt wird.

11. Ein Verfahren nach einem der vorstehenden Ansprüche, worin das Polyamin ein solches der allgemeinen Formel (IV):
NH₂(CH₂)_{q}-[NH(CH₂)_{q}]ᵣNH₂ (IV)
ist, in welcher q eine ganze Zahl mit einem Wert von 2 bis 4 bedeutet und r Null oder eine ganze Zahl mit einem Wert von 1 bis 10 ist.

12. Ein Verfahren nach Anspruch 11, worin das Polyamin Diethylentriamin ist.

13. Ein Verfahren nach einem der vorstehenden Ansprüche, worin das Additiv in einer weiteren Stufe hydriert ist.

14. Eine Treibstoffzusammensetzung, enthaltend einen Hauptbestandteil eines Treibstoffs und eine kleinere Menge eines Additivs, hergestellt durch ein Verfahren, wie es in irgendeinem der vorstehenden Ansprüche beansprucht wird.

## Revendications

1. Procédé pour la production d'un additif approprié à une incorporation dans une huile lubrifiante ou un carburant, procédé qui consiste à faire réagir une polyamine aliphatique avec un composé de formule (I) 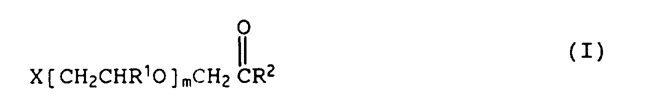 dans laquelle X représente un radical dérivé d'un nucléophile;
R¹ représente un atome d'hydrogène, un radical alkyle ou aryle;
R² représente un radical alkyle ou aryle;
m est un nombre compris entre 1 et 40; et
lorsque m est supérieur à un, chaque motif [CH₂CHR¹O] est identique ou différent à condition que lorsque R¹ représente un atome d'hydrogène, les motifs [CH₂CH₂O] ne constituent pas plus de 50% en poids du nombre total des motifs [CH₂CHR¹O].

2. Procédé selon la revendication 1, dans lequel dans la formule (I), X représente OH, SH, OR³, SR³, -NR⁴R⁵ ou un alcanoate, R³ représentant un radical hydrocarbyle et R⁴ et R⁵ représentant indépendamment un atome d'hydrogène ou un radical hydrocarbyle.

3. Procédé selon l'une des revendications 1 ou 2, dans lequel R¹ dans la formule (I) représente un radical méthyle ou éthyle.

4. Procédé selon l'une des revendications précédentes, dans lequel le composé de formule (I) est obtenu par oxydation du composant de formule (II):
X[CH₂CHR¹O]ₘCH₂CHR²(OH) (II)
dans laquelle X, R¹, R² et m sont tous tels que définis dans la revendication 1 à condition que lorsque R¹ est H, les motifs [CH₂CH₂O] ne constituent pas plus de 50% en poids du nombre total des motifs [CH₂CHR¹O].

5. Procédé selon la revendication 4, dans lequel le composé de formule (II) est oxydé par du dichromate.

6. Procédé selon la revendication 5, dans lequel le traitement au dichromate est réalisé à une température comprise entre 25 et 100°C.

7. Procédé selon la revendication 4, dans lequel le composé de formule (II) est oxydé par déshydrogénation catalytique.

8. Procédé selon la revendication 4, dans lequel le composé de formule (II) est oxydé par traitement avec un gaz contenant de l'oxygène moléculaire.

9. Procédé selon l'une des revendications précédentes, dans lequel le rapport de la polyamine au composé de formule (II) est compris entre 0,2 à 2,5:1.

10. Procédé selon la revendication 1, dans lequel on fait réagir un composé de formule (I) obtenu par oxydation d'un composé de formule (II) avec une polyamine aliphatique en employant un rapport molaire de la polyamine au polyéther compris entre 0,2 et 2,5 moles de polyamine par mole de polyéther.

11. Procédé selon l'une des revendications précédentes, dans lequel la polyamine a la formule générale (IV):
NH₂(CH₂)_{q} [NH(CH₂)_{q}]ᵣNH₂ (IV)
dans laquelle q est un nombre entier compris entre 2 et 4 et r est zéro ou un nombre entier compris entre 1 et 10.

12. Procédé selon la revendication 11, dans lequel la polyamine est la diéthylènetriamine.

13. Procédé selon l'une des revendications précédentes, dans lequel l'additif est hydrogéné dans une étape supplémentaire.

14. Composition de carburant comprenant une proportion majeure d'un carburant et une quantité mineure d'un additif préparé à l'aide d'un procédé selon l'une des revendications précédentes.
